(19) Europäisches Patentamt
European Patent Office
Office européen des brevets

(11) Veröffentlichungsnummer: **0 264 066**
**A2**

(12) # EUROPÄISCHE PATENTANMELDUNG

(21) Anmeldenummer: 87114698.1

(22) Anmeldetag: 08.10.87

(51) Int. Cl.⁴: A61K 7/06 , A61K 7/08 , A61K 7/48

(30) Priorität: 09.10.86 DE 3634417

(43) Veröffentlichungstag der Anmeldung: 20.04.88 Patentblatt 88/16

(84) Benannte Vertragsstaaten: CH DE FR GB LI

(71) Anmelder: **HOECHST AKTIENGESELLSCHAFT**
**Postfach 80 03 20**
**D-6230 Frankfurt am Main 80(DE)**

(72) Erfinder: **Hofinger, Manfred, Dr.**
**Rossfeldstrasse 7a**
**D-8269 Burgkirchen(DE)**
Erfinder: **Reng, Alwin**
**Im Schulzehnten 22**
**D-6233 Kelkheim Taunus(DE)**
Erfinder: **Quack, Jochen Meinhard, Dr.**
**Wilhelm-Reuter-Strasse 16**
**D-6239 Eppstein/Taunus(DE)**

(54) **Kosmetische Haar- und Hautbehandlungsmittel.**

(57) Haar-und Hautbehandlungsmittel mit einem Gehalt einer quartären Ammonium-Verbindung der Formel

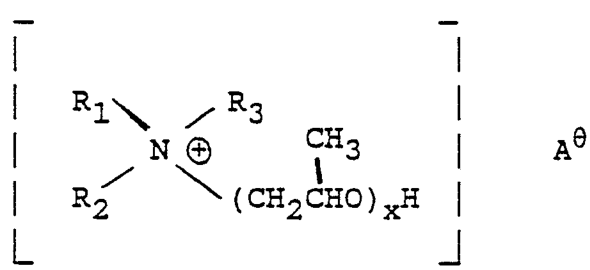

worin $R_1$ und $R_2$ gleich oder verschieden sein können und $C_8$-$C_{22}$-Alkyl oder $C_8$-$C_{22}$-Alkenyl, $R_3$ $C_1$-$C_3$-Alkyl, x eine Zahl von 1 bis 3 und $A^\theta$ das Anion einer $C_2$-$C_6$-Carbonsäure, die 1 bis 3 Hydroxyl-Gruppen enthalten kann, oder das Hydrogenphosphat-Anion bedeuten.

EP 0 264 066 A2

## Kosmetische Haar-und Hautbehandlungsmittel

Die Humanhaare werden durch wechselnde exogene Einflüsse geschädigt. Die Schädigung der Haarstruktur kann physikalisch, z.B. durch Kämmen, Bürsten und Toupieren oder beim Haarewaschen erfolgen. Eine weitere Schädigung wird beim Bleichen, Färben oder Dauerwellen der Haare beobachtet. Auch mikrobiologische Einflüsse können die Haarstruktur negativ beeinflussen. Um diese Einflüsse zu minimieren, ist es üblich, Haarbehandlungsmittel in Form von Haarnachspülmitteln oder Konditionierungsmitteln zu verwenden. Bei Anwendung dieser Präparate wird außerdem die Kämmbarkeit der nassen und trockenen Haare wesentlich erleichtert und gleichzeitig die statische Aufladung verringert. Diese kosmetischen Haarbehandlungsmittel können in wäßriger oder wäßrig-alkoholischer Lösung angewandt werden oder auch in Form von Emulsionen und/oder Suspensionen.

Es ist bekannt, daß zur Herstellung solcher kosmetischen Haarbehandlungsmittel am besten kationische Verbindungen, im allgemeinen quartäre Ammoniumverbindungen geeignet sind. Diese Tenside besitzen eine hohe Substantivität zum Haar und der entstehende mono-bzw. multimolekulare Film ergibt die gewünschten kosmetischen Eigenschaften. Die am meisten zur Herstellung von Haarbehandlungsmitteln verwendeten kationischen Tenside gehören in die Gruppen der Alkyltrimethylammoniumchloride bzw. -bromide, zu den Dialkyldimethylammoniumchloriden oder zu den Alkyldimethylbenzylammoniumchloriden.

Daneben werden auch noch wasserlösliche kationische Polymere allein oder in Kombination mit den vorgenannten quartären Ammoniumverbindungen eingesetzt.

Ein Großteil dieser verwendeten kationischen Tenside besitzt jedoch eine Reihe von Nachteilen, wie z.B. zu schwache Kämmbarkeit des nassen Haares, Beschwerung bzw. Akkumulation auf dem Haar, ungünstige dermatologische bzw. toxikologische Eigenschaften und schwierige Rezeptierung d.h. nicht ausreichende Erhöhung der Konsistenz.

Es wurde nun gefunden, daß überraschenderweise mit den nachstehend aufgeführten oxpropylierten quartären Ammoniumverbindungen Mittel hergestellt werden können, die diese Nachteile nicht besitzen. Gegenstand der Erfindung sind somit Behandlungsmittel für Haare und auch für die Haut mit einem Gehalt einer quartären Ammonium-Verbindung der Formel

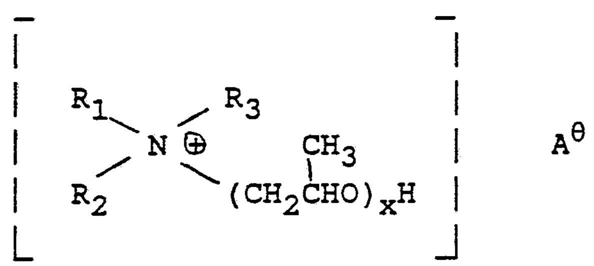

worin $R_1$ und $R_2$ gleich oder verschieden sein können und $C_8$-$C_{22}$-Alkyl oder $C_8$-$C_{22}$-Alkenyl, $R_3$ $C_1$-$C_3$-Alkyl, x eine Zahl von 1 bis 3 und $A^{\theta}$ das Anion einer $C_2$-$C_6$-Carbonsäure, die 1 bis 3 Hydroxyl-Gruppen enthalten kann, oder das Hydrogenphosphat-Anion bedeuten.

Vorzugsweise bedeuten $R_1$ und $R_2$ $C_{12}$-$C_{18}$-Alkyl, $R_3$ Methyl, x eine Zahl von 1,5 bis 2,5 $A^{\theta}$ das Anion der Milchsäure, Citronensäure oder Weinsäure. Die quartären Ammonium-Verbindungen der obigen Formel lassen sich herstellen analog zu dem in US-PS 4 409 109 beschriebenen Verfahren.

Mit diesen oxpropylierten quartären Ammoniumverbindungen lassen sich Haarbehandlungsmittel herstellen, die daneben noch die üblicherweise verwendeten Komponenten, wie Konsistenzgeber, Fettkomponenten, Konservierungsmittel, Parfümöle, Farbstoffe, Lichtschutzmittel u.a. kosmetische Wirkstoffe emulgiert oder dispergiert in Wasser oder gelöst in Alkoholen enthalten. Die beschriebenen quartären Ammoniumverbindungen haben bei der Anwendung in konditionierenden Haarnachspülmitteln, Haarfestigern oder Fönlotionen folgende Vorteile.

## 1. Verbesserung der Naßkämmbarkeit

Die Prüfung der Kämmbarkeit von nassem Haar erfolgt in-vivo an mindestens 6 Testpersonen mit möglichst vorgeschädigtem Haar.

Die Durchführung erfolgt durch praxisgerechtes zweimaliges Waschen der Haare der Versuchspersonen unter standardisierten Bedingungen und anschließendes leichtes Trocknen. Das Haar der Probanden wird in der Mitte des Kopfes gescheitelt und mit jeweils 10 ml wäßriger Lösung behandelt, die die quart. Ammoniumverbindungen in einer Konzentration von 0,1 % enthält. Die Einwirkungszeit bei Raumtemperatur beträgt 5 Minuten. Anschließend wird mit handwarmen Leitungswasser 2 Minuten gespült und entsprechend der Praxis das Haar mit Handtüchern von der überschüssigen Wassermenge befreit. Unter Verwendung von 2 gleichen Kämmen werden die zu vergleichenden Seiten synchron gekämmt und der auftretende Wiederstand festgestellt. Es zeigte sich, daß bei Verwendung der erfindungsgemäßen Substanzen im Vergleich zu den handelsüblichen quart. Ammoniumverbindungen eine sehr gute Kämmbarkeit des nassen Humanhaares gegeben ist.

Auch die Kämmbarkeit des anschließend getrockneten Haares ist sehr gut. Das gleiche gilt für die sogenannten Frisiereigenschaften der Haare. (siehe Tabelle 1)

Tabelle 1

| | Kämmbarkeit nasses Haar | Kämmbarkeit trockenes Haar | Antistatik | Griff der Haare |
|---|---|---|---|---|
| Cetyltrimethylammoniumchlorid (Vergleich) | 3 | 3 | 4 | 2 |
| Stearyldimethylbenzylammonium-chlorid (Vergleich) | 2 | 3 | 2 | 3 |
| Distearylmethylpoly-(oxpropyl)-ammoniumlactat (erfindungsgemäß) | 4 | 4 | 3-4 | 4 |
| ohne quart. Ammoniumverbindung | 0 | 1 | 1 | 1 |

4 = sehr gut
3 = gut
2 = mäßig
1 = schlecht
0 = sehr schlecht

## 2. Schaumverhalten bei der Anwendung

Ein weiterer Vorteil der oxpropylierten quartären Ammoniumverbindungen ist ihre Schaumfreiheit in wäßriger Verdünnung. Von besonderem Vorteil ist dies bei der Anwendung in Haarkonditionierungsmitteln, da eine hohe Schaumbildung dem Verbraucher das Vorhandensein von Restmengen Shampoos vortäuscht, was wiederum zu einem zeitaufwendigen und störenden verstärkten Ausspülen der Haarkur nach deren Anwendung führt. Beim Einsatz der oxpropylierten quartären Ammoniumverbindungen wird im Vergleich zu dem praxisüblichen Cetyltrimethylammoniumchlorid keine Schaumwirkung beobachtet.

In-vitro läßt sich das Schaumverhalten auch nach der sogenannten Schlagschaummethode entsprechend der DIN 53 902 bestimmen. Eine 0,1 % Aktivsubstanz enthaltende wäßrige Lösung mit einer Wasserhärte von 20 °dH wird bei 20°C unter standardisierten Bedingungen 25 mal geschlagen. Das Schaumvolumen einer Cetyltrimethylammoniumchloridlösung beträgt hierbei 740 $cm^3$, während die oxpropylierte quart. Ammoniumverbindungen keinerlei Schaumwirkung zeigt.

## 3. Einfache Rezeptierung

Die bevorzugte Form von Haarbehandlungsmitteln ist eine Dispersion, enthaltend die quartären Ammoniumverbindungen und zusätzliche Konsistenzgeber, wie z.B. Cetylalkohol und/oder Stearylalkohol. Durch die Verwendung dieser Konsistenzgeber wird eine praxisgerechte Viskosität erreicht, die einerseits die Dosierung erleichtert und anderseits eine bessere Verteilung auf den Haaren ermöglicht. Die Einarbeitung dieser Substanzen ist technologisch aufwendig und die Endviskosität stellt sich z.T. erst nach längerer Zeit auf das Maximum ein.

Bei den erfindungsgemäßen oxpropylierten quartären Ammoniumverbindungen wurde gefunden, daß es möglich ist, mittel-bis hochviskose Haarspülmittel herzustellen, ohne daß zusätzliche Konsistenzgeber erforderlich sind. Beispielsweise wurden bie 20°C mit einem Brookfield-Viskosimeter folgende Viskositätswerte in Abhängigkeit von der Konzentration gefunden:

1 % Wirksubstanz gelöst in Wasser: 1140 mPas
2 % Wirksubstanz gelöst in Wasser: 2050 mPas
3 % Wirksubstanz gelöst in Wasser: 2180 mPas
5 % Wirksubstanz gelöst in Wasser: 2810 mPas

Die erfindungsgemäß zu verwendenden oxpropylierten quartären Ammoniumverbindungen können in an sich bekannter Weise in die Haarspülmittel eingearbeitet werden. Dabei werden 0,5 - 10 % der oxpropylierten quartären Ammoniumverbindungen allein oder in Kombination mit anderen quartären Ammoniumverbindungen zusammen mit dem üblicherweise verwendeten Farbstoffen, Duftstoffen, haarkosmetischen Wirkstoffen und Konservierungsmitteln in Wasser bzw. Wasser/Alkohol-Mischungen dispergiert.

Auch in haarkosmetischen Präparaten, die nach der Anwendung nicht mit Wasser ausgespült werden, können die erfindungsgemäßen Substanzen eingearbeitet werden. Dies betrifft beispielsweise Haarwässer, die etwa 0,05 bis 0,1 % der genannten oxpropylierten quartären Ammonium-Verbindungen enthalten können, oder Haarfestiger, Fönwellmittel in flüssiger, cremeförmiger Zubereitung oder als Aerosole mit einem Gehalt von etwa 0,1 bis 1 % dieser Verbindungen.

Prinzipiell ist die Verwendung der erfindungsgemäßen Substanzen auch zur Herstellung von kosmetischen Emulsionen zur Pflege der Haut möglich. Durch den substantiven Charakter der Substanzen wird ein verbessertes Hautgefühl nach der Anwendung erzielt und gleichzeitig aufgrund der Emulgierwirkung eine stabilisierende Wirkung in den Fertigpräparaten erreicht.

Die Anwendung der oxpropylierten quartären Ammoniumverbindungen wird in den nachstehenden Beispielen weiter erläutert. Sofern nicht anders angegeben, beziehen sich alle Mengen auf das Gewicht.

## Beispiel 1

**Haarnachspülmittel**

3,0 % Distearylmethylpoly-$(oxypropyl)_{2-3}$ammoniumlactat
1,5 % Cetyl-stearylalkohol + 3 Mol Ethylenoxid
3,0 % Cetylalkohol
0,3 % Parfümöl
ad 100 % Wasser

Beispiel 2

**Haarbehandlungsmittel ohne Konsistenzgeber**

3,0 % Distearylmethylpoly-(oxypropyl)$_{2-3}$ammoniumlactat
0,2 % Parfümöl
ad 100 % Wasser

Beispiel 3

**Fönwellmittel**

0,2 % Distearylmethylpoly-(oxypropyl)$_{2-3}$ammoniumlactat
15 % Isopropylalkohol
0,2 % Polyvinylpyrrolidon
0,1 % Parfümöl
ad 100 % Wasser

Beispiel 4

**Haarnachbehandlungsmittel**

2,0 % Distearylmethylpoly-(oxypropyl) $_{2-3}$ammoniumlactat
1,0 % Pentaoxäthylammoniumlactat
3,5 % Cetylalkohol
ad 100 % Wasser

Beispiel 5

**Haarnachspülmittel**

2,5 % Distearylmethylpoly-(oxypropyl)$_{2-3}$ammoniumlactat
0,5 % Behenyltrimethylammoniumchlorid
3,0 % Cetylalkohol
ad 100 % Wasser

Beispiel 6

**Aerosolförmiges Haarnachbehandlungsmittel**

0,1 % Distearylmethylpoly-(oxypropyl) $_{2-3}$ammoniumlactat
0,1 % Cetyltrimethylammoniumbromid
0,7 % Cetylstearylalkohol
ad 100 % Wasser

Abfüllung der obigen Mischung in bekannter Weise mit einem Treibmittel in Aerosolbehälter.

Beispiel 7

**Flüssiges Hautpflegemittel**

1,0 % Distearylmethylpoly-(oxypropyl)$_{2-3}$ ammoniumlactat
3,0 % Tetraethylenglykol-polyglyceryl-di-stearat
10,0 % Paraffinöl
10,0 % Isopropylpalmitat
0,1 % Parfümöl
ad 100 % Wasser

Beispiel 8

**Cremeförmiges Hautpflegemittel**

2,0 % Distearylmethylpoly-(oxypropyl)$_{2-3}$ammoniumlactat
6,0 % Tetraethylenglykol-polyglyceryl-di-stearat
15,0 % Paraffinöl
5,0 % Isopropylmyristat
ad 100 % Wasser

## Ansprüche

1. Haar-und Hautbehandlungsmittel mit einem Gehalt einer quartären Ammonium-Verbindung der Formel

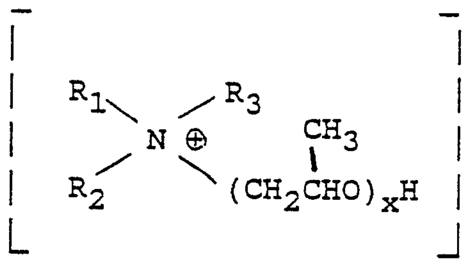

$A^{\theta}$

worin $R_1$ und $R_2$ gleich oder verschieden sein können und $C_8$-$C_{22}$-Alkyl oder $C_8$-$C_{22}$-Alkenyl, $R_3$ $C_1$-$C_3$-Alkyl, x eine Zahl von 1 bis 3 and $A^{\theta}$ das Anion einer $C_2$-$C_6$-Carbonsäure, die 1 bis 3 Hydroxyl-Gruppe enthalten kann, oder das Hydrogenphosphat-Anion bedeuten.

2. Haarbehandlungsmittel nach Anspruch 1 mit einem Gehalt einer quartären Ammonium-Verbindung der angegebenen Formel, wobei $R_1$ und $R_2$ $C_{12}$-$C_{18}$-Alkyl, $R_3$ Methyl, x eine Zahl von 1,5 bis 2,5 und $A^{\theta}$ das Anion der Milchsäure, Citronensäure oder Weinsäure bedeuten.